# EUROPEAN PATENT APPLICATION

(11) **EP 4 806 553 A1**
(43) Date of publication of application: **16.09.2026**
(21) Application number: 25163217.0
(22) Date of filing: 12.03.2025
(51) Int. Cl.: B01L 3/00, G01N 33/543

(54) **METHOD FOR FABRICATING AN ANALYSIS DEVICE, ANALYSIS DEVICE, USE THEREOF FOR DETECTING ANALYTES FROM A FLUID, AND APPARATUS FOR FABRICATING THE ANALYSIS DEVICE**

(71) Applicant: Technische Universität München, in Vertretung des Freistaates Bayern, 80333 München (DE)
(72) Inventor: DESTGEER, Ghulam, 81827 Munich (DE); YANG, Yimin, 80939 Munich (DE); AKHTAR, Muhammad Usman, 81241 Munich (DE)
(74) Representative: Lucke, Andreas

(57) **Abstract**

A method for fabricating an analysis device is provided. The analysis device comprises a capillary tube and hydrogel elements on an inner surface of the capillary tube. The hydrogel elements comprise a hydrogel and a binding agent for immobilizing an analyte from a fluid. The method comprises providing parallel flows of fluids in the capillary tube. The parallel flows of fluids comprise a first flow of a first fluid on the inner surface of the capillary tube. The first fluid comprises a hydrogel precursor and the binding agent. The method further comprises stopping the parallel flows of fluids in the capillary tube. The method also comprises providing, through a lithographic mask, blue or UV light in the capillary tube to photopolymerize at least a portion of the hydrogel precursor comprised in the first fluid, thereby forming, on the inner surface of the capillary tube, the hydrogel elements comprising the hydrogel and the binding agent.

## Description

### FIELD OF THE DISCLOSURE

The present disclosure relates to a method, an apparatus, and an analysis device for fabricating and utilizing capillary tubes with hydrogel elements for analyte detection. Specifically, the present disclosure pertains to the formation of hydrogel elements on the inner surface of capillary tubes using photopolymerization, enabling efficient immobilization and detection of analytes from fluids.

### BACKGROUND

For the compartmentalization of aqueous specimen, analysis devices in the form of microwell plates are commonly applied. Microwell plates typically provide up to 1536 compartmentalized spaces in the form of microwells, each typically providing a volume in a range of tens of nanoliters up to several milliliters.

The functioning of a respective analysis device involves introducing a sample fluid containing the analytes into the wells. Binding agents are placed within the wells and interact with the analytes, immobilizing them on the surface. Subsequent washing steps remove unbound substances, and detection reagents are added to produce a measurable signal, such as a color change, fluorescence, or luminescence, indicating the presence and quantity of the analytes.

Respective analysis devices find application in clinical diagnostics, pharmaceutical research, and environmental monitoring. Technologically, they enable precise and sensitive detection of biomolecules, facilitating advancements in medical research and personalized medicine. Scientifically, these devices are valuable tools in studying biological processes, disease mechanisms, and the effects of therapeutic agents.

### SUMMARY OF THE DISCLOSURE

It is an object of this invention to provide improvements in relation to analysis devices, and, in particular, to provide an improved method of producing an analysis device, an apparatus for the production, and the resulting improved analysis device.

According to an aspect of the disclosure, a method for fabricating an analysis device is provided. The analysis device comprises a capillary tube and hydrogel elements on an inner surface of the capillary tube. The hydrogel elements comprise a hydrogel and a binding agent for immobilizing an analyte from a fluid. The method comprises providing parallel flows of fluids in the capillary tube. The parallel flows of fluids comprise a first flow of a first fluid on the inner surface of the capillary tube. The first fluid comprises a hydrogel precursor and the binding agent. The method further comprises stopping the parallel flows of fluids in the capillary tube. The method also comprises providing, through a lithographic mask, blue or UV light in the capillary tube to photopolymerize at least a portion of the hydrogel precursor comprised in the first fluid, thereby forming, on the inner surface of the capillary tube, the hydrogel elements comprising the hydrogel and the binding agent.

The method produces multiple hydrogel elements for capturing a fluid introduced into the capillary tube. More specifically, when the fluid is introduced into the capillary tube, the hydrogel of each of the hydrogel elements absorbs and retains a portion of the fluid, which is typically on the order of 1 mm³ or less and may be as small as a few nanoliters. With the binding agent comprised in the hydrogel element, each of the hydrogel elements provides the functionality to act as a detector for a specific substance that the binding agent is adapted to selectively immobilize. In other words, the disclosed analysis device comprising the capillary tube and the hydrogel elements provides the function to detect analytes in a number of detectors provided by the hydrogen elements in parallel. In the context of this disclosure, a respective device is also referred to as a "lab on a capillary".

The disclosed method produces the capillary device with an improved spatial resolution, or an improved spatial control, respectively, over the sizes and shapes of the hydrogel elements. In particular, the resolution/control is improved compared to conventional techniques such as spraying a polymer for forming a hydrogel onto a workpiece or applying a polymer for forming a hydrogel onto a workpiece using a syringe. The improved resolution/control is a consequence both of the process step of stopping the parallel flows of liquids in the capillary tube, which generates a defined pattern of the fluids from the parallel flows of fluids in the capillary tube, and the use of a photopolymerization through a lithographic mask, resulting in a spatial resolution significantly improved over the aforementioned, conventional techniques. By halting the flow, the method ensures that the hydrogel precursor and the binding agent are positioned correctly before the photopolymerization step, leading to accurate patterning of the hydrogel structures. The use of a lithographic mask allows for precise spatial control over where the light is applied, enabling the creation of patterned hydrogel elements. This precision is beneficial for creating complex structures such as for multiplexed assays and high-sensitivity detection.

As compared to a conventional device, wherein the entire inner surface of the capillary is used for the detection of analytes, the compartmentalization into multiple hydrogel elements improves the detection accuracy, for example in an assay performed using the binding agent comprised in the hydrogel elements.

Moreover, the photopolymerization results in the formation of a covalently cross-linked hydrogel, which is particularly beneficial in the use of an analysis device, since the covalent cross-linking improves both the adhesion of the hydrogel elements to the capillary, and the absorption and retention of liquid for and during the analysis.

The porosity and hydrophilicity of the hydrogel elements may also be used to absorb the fluid with the analyte in a reversible manner, i.e., by rinsing the capillary with a suitable liquid, the hydrogen elements may be depleted of the fluid with the analyte. Thereafter, a new fluid to be analyzed may be introduced into the capillary tube and thus into the hydrogen elements.

The compartmentalization into the hydrogel elements gives rise to an improved signal amplification, for example in a bioassay, due to an improved enzymatic amplification reaction achieved by the compartmentalization.

The method may be performed in a production facility at ambient conditions, resulting in the fabrication of analysis devices of high quality. This is because, when the parallel flows of fluids are provided and stopped in the capillary tube, and when the blue or UV light is provided to photopolymerize the (at least one portion of the) hydrogel precursor, the inner surface of the capillary tube and the fluids as well as the hydrogel precursor and the binding agent comprised therein are enclosed in a fluid-tight manner. Only when the capillary tube is exchanged for a new capillary tube (e.g., for subsequent fabrication of a further analysis device), the enclosure is broken. However, at this stage, the produced analysis device is typically still filled with the remaining fluids of the parallel flows of fluids, or with a rinse solution that, after the photopolymerizing, the fluids of the parallel flows of fluids are exchanged with, preventing unwanted particular ingress, e.g., from air. Possible contaminations of the new capillary tube, in the subsequent fabrication of the further analysis device, may be rinsed out by applying a rinsing solution through the new capillary tube before the process steps of the disclosed method are performed.

The option to work in a production facility at ambient conditions, i.e., without the need for clean room facilities, is an advantage over conventional techniques, in particular over those, in which a capillary tube is exposed to air during the conventional technique.

The binding agent may be a binding agent for selectively immobilizing the analyte from the fluid.

According to some embodiments, the parallel flows of fluids comprise a second flow of a second fluid, wherein a weight percentage of the hydrogel precursor is smaller in the second fluid than in the first fluid.

According to a respective embodiment, the parallel flows of fluids are provided in the capillary tube such that, in a cross section of the capillary tube, the first flow of the first fluid surrounds at least a portion of the second flow of the second fluid. According to a respective embodiment, in the process step of forming the hydrogel elements comprising the hydrogel and the binding agent, the hydrogel elements are formed to surround a portion of an inner cross-sectional area of the capillary tube, said portion of the inner cross-sectional area of the capillary tube corresponding to said at least one portion of the second flow of the second fluid.

Respective embodiments ensure that the hydrogel elements are generated along the inner surface of the capillary tube, increasing the area of an interface between (in particular, each of) the hydrogen elements and said inner surface, thus improving the adhesion of the hydrogel elements to the inner surface of the capillary tube. This is an advantage over a conventional analysis device wherein a detection element only adheres to, or only has an interface with, respectively, a point on or a small area of a substrate (such as a tube), resulting in a larger risk for the detection elements to detach from the substrate.

Also, the analysis device wherein the hydrogel elements surround a portion of an inner cross-sectional area of the capillary tube enlarges the surface of the hydrogel element exposed to the fluid, when the fluid is introduced into the capillary tube. This improves the absorption of the fluid into the hydrogen element.

According to an embodiment, the method involves providing parallel flows of fluids in the capillary tube such that the first flow of the first fluid surrounds the second flow of the second fluid.

In the context of this disclosure, "the first flow of the first fluid surrounds the second flow of the second fluid" may refer to the arrangement where the first fluid forms an outer layer or sheath around the second fluid, which flows as a core within the capillary tube. This configuration is typically achieved using co-flow techniques, where the first fluid is introduced at a higher flow rate or pressure to envelop the second fluid.

According to an embodiment, in the process step of forming the hydrogel elements comprising the hydrogel and the binding agent, the hydrogel elements are formed to encircle the portion of the inner cross-sectional area of the capillary tube.

This configuration maximizes the surface area available for interaction with the analyte, thereby enhancing the efficiency of analyte capture and immobilization. Additionally, this arrangement can improve the structural integrity and stability of the hydrogel elements within the capillary tube.

In the context of this disclosure, the term "encircle" is to be understood such that a closed curve, extending inside of the (or through the, respectively) hydrogel elements, exists, fully encircling the portion of the inner cross-sectional area of the capillary tube. The term "surround", in contrast, is more general and does not require the portion of the inner cross-sectional area of the capillary tube to be fully encircled by the hydrogel elements, but includes embodiments wherein the hydrogel element is arranged on two sides or on three sides (e.g., according to a cross section through the capillary tube) of the portion of the inner cross-sectional area of the capillary tube.

According to some embodiments, the weight percentage of the hydrogel precursor in the second fluid is smaller than the weight percentage of the hydrogel precursor in the first fluid at least by a factor of two, or at least by a factor of five, or at least by a factor of ten, or at least by a factor of twenty.

By controlling the concentration of the hydrogel precursor in different fluid streams, the method allows for the precise patterning of hydrogel elements, which can be functionalized with specific binding agents.

Alternatively, or in addition, the second fluid may not comprise the hydrogel precursor.

The advantage of not comprising the hydrogel precursor in the second fluid is that it allows for the creation of clear, well-defined interfaces between hydrogel and non-hydrogel regions. This can be particularly useful for compartmentalization within the capillary tube, enabling precise control over reaction environments and preventing cross-contamination between different assay regions.

According to some embodiments, a density of the second fluid equals a density of the first fluid within 30% of the density of the first fluid, in particular, within 20% of the density of the first fluid or within 10% of the density of the first fluid.

By ensuring that the fluids' densities are similar, the method stabilizes the parallel flow, reducing the risk of mixing or turbulence that could compromise the formation of hydrogel elements. This is particularly beneficial in maintaining a clear interface between the fluids, which improves the spatial control during photopolymerization of the hydrogel elements. It ensures that the hydrogel precursor in the first fluid is accurately positioned and polymerized, leading to well-defined hydrogel elements that enhance the accuracy and reliability of the analysis device. This precise control over fluid dynamics results in more reproducible immobilization of analytes and improves reliability of the analysis device.

In a further implementation of the method, the first fluid and the second fluid are adapted to be miscible.

When the fluids are miscible, they can form a particularly stable and uniform flow, which is beneficial for the subsequent steps of stopping the flow and photopolymerizing the hydrogel precursor. This results in the formation of well-defined hydrogel elements on the inner surface of the capillary tube, enhancing the precision and effectiveness of the analysis device.

According to an embodiment, the method further comprises, after providing the blue or UV light in the capillary tube to photopolymerize the at least one portion of the hydrogel precursor comprised in the first fluid: providing subsequent parallel flows of fluids in the capillary tube, said subsequent parallel flows of fluids comprising a first subsequent flow of a first subsequent fluid on the inner surface of the capillary tube, wherein the first subsequent fluid comprises a subsequently-applied hydrogel precursor and an additional binding agent, wherein the additional binding agent is adapted to immobilize an additional analyte from the fluid, the additional analyte being different from the analyte; stopping the subsequent parallel flows of fluids in the capillary tube; and providing, through a subsequently-applied lithographic mask, blue or UV light in the capillary tube to photopolymerize at least a portion of the subsequently-applied hydrogel precursor comprised in the first subsequent fluid, thereby forming, on the inner surface of the capillary tube, additional hydrogel elements, the additional hydrogel elements comprising a subsequently-formed hydrogel and the additional binding agent, wherein the subsequently-formed hydrogel is formed, using the photopolymerization, from said at least one portion of the subsequently-applied hydrogel precursor.

Providing subsequent parallel flows of fluids in the capillary tube allows for the creation of multiple, distinct hydrogel regions within the same capillary tube. This enhances the multiplexing capability of the device, enabling the simultaneous detection of different analytes within a single capillary tube.

The inclusion of an additional binding agent adapted to immobilize a different analyte allows for the detection of multiple targets within the same assay, significantly increasing the versatility and utility of the analysis device. This is particularly advantageous for applications requiring the simultaneous monitoring of multiple biomarkers.

In other words, the formation of additional hydrogel elements with different binding agents within the same capillary tube enables the device to capture and analyze multiple analytes simultaneously, improving the efficiency and effectiveness of the assay.

In other words, well-defined, isolated compartments are created within the same capillary tube, each targeting a different analyte. The method thus fabricates an analysis device which is highly versatile and efficient for multiplexed analyte detection.

Optional, additional and/or preferred process steps and features described above in the context of providing the parallel flows of fluids may be applied correspondingly when providing the subsequent parallel flows of fluids.

Optional, additional and/or preferred process steps and features described above in the context of stopping the parallel flows of fluids in the capillary tube may be applied correspondingly when stopping the subsequent parallel flows of fluids in the capillary tube.

Optional, additional and/or preferred process steps and features described above in the context of providing the blue or UV light in the capillary tube to photopolymerize the at least one portion of the hydrogel precursor comprised in the first fluid may be applied correspondingly when providing the blue or UV light in the capillary tube to photopolymerize the at least one portion of the subsequently-applied hydrogel precursor comprised in the first subsequent fluid.

According to an embodiment, the lithographic mask and the subsequently-applied lithographic mask are arranged at different positions along a parallel-flow direction, said parallel-flow direction being the flow direction of the parallel flows of fluids when the parallel flows of fluids are provided in the capillary tube.

The subsequently-applied lithographic mask may be an additional lithographic mask different from the lithographic mask.

Respective embodiments allow for using a fabrication apparatus with fixed positions of the lithographic mask and the additional lithographic mask, thus avoiding the need to move any of the respective components during the method, which can improve the resolution of the method.

A same lithographic mask may be used as the lithographic mask and as the subsequently-applied lithographic mask.

In respective embodiments, in the process step of providing the blue or UV light in the capillary tube through the lithographic mask, the same lithographic mask may be arranged at a first position relative to the capillary tube; in the process step of providing the blue or UV light in the capillary tube through the subsequently-applied lithographic mask, the same lithographic mask may be arranged at a second position relative to the capillary tube, the second position being different from the first position. In respective embodiments, the method may further comprise, in between the process step of providing the blue or UV light in the capillary tube through the lithographic mask and the process step of providing the blue or UV light in the capillary tube through the subsequently-applied lithographic mask, moving at least one of the capillary tube and the same lithographic mask such that the same lithographic mask moves from the first position to the second position.

Respective embodiments allow for the generation of an analysis device wherein the hydrogel elements have a reduced spacing from the additional hydrogel elements, thus improving the use of space of the inner surface of the capillary tube.

According to an embodiment, the method further comprises, between the process step of providing the blue or UV light in the capillary tube through the lithographic mask and the process step of providing the subsequent parallel flows of fluids in the capillary tube, removing the first fluid from the capillary tube.

In respective embodiments, removing the first fluid from the capillary tube may comprise rinsing the capillary tube with a rinsing liquid, wherein a weight percentage of the hydrogel precursor is smaller in the rinsing liquid than in the first fluid.

The smaller weight percentage of the hydrogel precursor in the rinsing liquid may be characterized by features similar to the ones described above in the context of the smaller weight percentage of the hydrogel precursor in the second fluid.

The subsequent parallel flows of fluids may comprise a second subsequent flow of a second subsequent fluid, a weight percentage of the subsequently-applied hydrogel precursor being smaller in the second subsequent fluid than in the first subsequent fluid. The subsequent parallel flows of fluids may be provided in the capillary tube such that the first subsequent flow of the first subsequent fluid surrounds at least a portion of the second subsequent flow of the second subsequent fluid. In respective embodiments, in the process step of forming the additional hydrogel elements comprising the subsequently-formed hydrogel and the additional binding agent, the additional hydrogel elements may be formed to surround a second portion of an inner cross-sectional area of the capillary tube, the second portion of the inner cross-sectional area of the capillary tube corresponding to said at least one portion of the second subsequent flow of the second subsequent fluid.

Optional, additional and/or preferred process steps and features described above in the context of the second flow of the second fluid may apply correspondingly to the second subsequent flow of the second subsequent fluid.

Optional, additional and/or preferred process steps and features described above in the context of the portion of the inner cross-sectional area of the capillary tube may apply correspondingly to the second portion of the inner cross-sectional area of the capillary tube.

The subsequently-formed hydrogel may comprise or may be a covalently cross-linked hydrogel. The method may further comprise forming the covalently cross-linked hydrogel in the process step of photopolymerizing the at least one portion of the subsequently-applied hydrogel precursor comprised in the first subsequent fluid.

The provision of the second portion of the inner cross-sectional area of the capillary tube has similar advantages as described above in the context of the provision of the portion of the inner cross-sectional area of the capillary tube.

The provision of a covalently cross-linked hydrogel as the subsequently-formed hydrogel has similar advantages as described above in the context of the provision of the covalently cross-linked hydrogel as the hydrogel of the hydrogel elements.

The hydrogel may comprise or be a covalently cross-linked hydrogel. The method may further comprise forming the covalently cross-linked hydrogel in the process step of photopolymerizing the at least one portion of the hydrogel precursor comprised in the first fluid.

The covalently cross-linked hydrogel (and/or the subsequently-formed hydrogel, respectively) may refer to a hydrogel network where the polymer chains are interconnected through covalent bonds. These bonds are strong chemical bonds that provide stability and robustness to the hydrogel structure, making it less likely to dissolve or degrade in the presence of fluids. This stability ensures that the hydrogel can effectively immobilize analytes and withstand the mechanical stresses of fluid flow within the capillary tube.

An inner cross-sectional area of the capillary tube may be no larger than 1 mm².

According to an embodiment, an inner cross-sectional area of the capillary tube has, along at least one direction, an extension of no more than 1 mm.

An inner cross-sectional area of the capillary tube may be circular or elliptical and no larger than 1 mm². In respective embodiments, optionally, the inner cross-sectional area of the capillary tube may be circular with a diameter of no more than 1 mm.

A smaller inner cross-sectional area of the capillary tube, or a smaller extension/width of the inner cross-sectional area, respectively, allows for the handling of very small volumes of fluid, which is beneficial for high-sensitivity assays and reduces the consumption of reagents. Additionally, it enhances the capillary action, facilitating the movement and retention of fluids within the capillary tube without the need for external pumps or instruments. This leads to more accurate and reliable immobilization of analytes, enhancing the sensitivity and specificity of the assay.

In other words, a small inner cross-sectional area allows for more precise control of fluid flow and enhances the capillary action, which is beneficial for the loading and unloading of solutions without the need for external instruments.

The hydrogel elements, when they are formed on the inner surface of the capillary tube, may be formed in direct physical contact with the inner surface of the capillary tube.

The hydrogel elements, when they are formed on the inner surface of the capillary tube, may be formed on an interlayer such that the interlayer is located between the hydrogel elements and the inner surface of the capillary tube, in particular, wherein the interlayer comprises at least one of a siloxane and a silane layer covalently bonded to the inner surface of the capillary tube.

An adhesive layer may be provided as the interlayer.

The interlayer may refer to a thin layer of material that is applied to the inner surface of the capillary tube before the hydrogel elements are formed. This interlayer serves as a bonding interface between the hydrogel elements and the capillary tube, enhancing the adhesion and durability of the hydrogel structures.

In other words, by incorporating an interlayer between the hydrogel elements and the inner surface, the method ensures that the hydrogel elements are securely attached, enhancing the robustness and reliability of the analysis device, resulting in a more stable and effective platform for immobilizing and analyzing analytes from fluid samples.

In particular, the inclusion of a siloxane or silane layer as the interlayer provides a robust and chemically stable interface between the hydrogel elements and the capillary tube, providing a strong adhesion and resistance to chemical degradation, ensuring that the hydrogel elements remain securely attached to the capillary surface even under challenging conditions.

The inner surface of the capillary tube may comprise or may be composed of glass.

The capillary tube may comprise or may be composed of glass.

Glass provides transparency, chemical inertness, and the ability to be precisely fabricated into various shapes and sizes, making it suitable for applications in analytical devices, particularly in microfluidic systems.

The method may further comprise, before the hydrogel elements are formed on the inner surface of the capillary tube, silanizating the inner surface of the capillary tube; wherein the hydrogel elements are formed on the silanizated inner surface of the capillary tube.

This treatment creates a layer of silane molecules on the surface, which provide functional groups for further chemical reactions during the photopolymerization step, ultimately resulting in the hydrogel elements being firmly attached to the surface of the capillary tube. Thus, the risk of detachment of the hydrogel elements from the inner surface of the capillary tube is reduced.

Silanizating the inner surface of the capillary tube may comprise: hydroxylating the inner surface of the capillary tube; and applying a silane to the hydroxylated inner surface of the capillary tube.

In the context of this disclosure, hydroxylating the inner surface of the capillary tube may refer to a chemical process that introduces hydroxyl groups (-OH) onto the inner surface of the capillary tube. This process can be achieved through various chemical treatments, such as exposure to a strong acid or base, which cleans and prepares the surface for subsequent modifications.

This step increases the surface reactivity, allowing for better adhesion of subsequent layers, such as silane, and improving the overall stability and functionality of the hydrogel elements formed on the surface.

In the context of this disclosure, applying a silane to the hydroxylated inner surface of the capillary tube may refer to the process of coating the hydroxylated surface with a silane compound. Silane compounds typically contain a silicon atom bonded to organic groups and reactive groups that can form covalent bonds with the hydroxyl groups on the surface.

Hydroxylating the inner surface of the capillary tube and applying a silane to the hydroxylated inner surface of the capillary tube function together to ensure that the inner surface of the capillary tube is properly prepared and functionalized for the formation of hydrogel elements. This preparation enhances the adhesion and stability of the hydrogel elements, resulting in an increased durability and performance of the analysis device, leading to more reliable and accurate detection of analytes.

The binding agent may comprise at least one of the following, or may be one of the following: a tag adapted to bind a capture antibody thereto, a capture antibody, and nanoparticles coated with a capture antibody.

In the context of this disclosure, a capture antibody may refer to a component that is specifically designed to bind to a particular analyte, such as a protein or biomolecule, from a fluid sample. Capture antibodies are typically monoclonal or polyclonal antibodies that have a high affinity for the target analyte, allowing for selective and efficient immobilization within the hydrogel matrix.

Capture antibodies provide high specificity and sensitivity in detecting target analytes, which is beneficial for accurate and reliable bioassays. This specificity reduces the likelihood of cross-reactivity and false positives, thereby enhancing the assay's precision. Additionally, the presence of capture antibodies within the hydrogel matrix can facilitate the creation of multiple, distinct detection zones within a single capillary tube, enabling parallel and/or multiplexed assays that can simultaneously detect analytes from a single fluid.

Nanoparticles pre-coated with a variety of different capture antibodies are commercially available. The potential of the method to integrate any of those nanoparticles precoated with a capture antibody enhances the versatility of the method and of the fabricated analysis device.

Nanoparticles have a high surface area to volume ratio, which allows for a greater number of capture antibodies to be immobilized on their surface. This increases the likelihood of capturing the target analyte, thereby improving the sensitivity and efficiency of the assay. Additionally, nanoparticles can enhance signal amplification, leading to more accurate and reliable detection of low-abundance analytes.

In particular, the binding agent may comprise a tag adapted to selectively bind a capture antibody thereto. Alternatively, or in addition, the binding agent may comprise a capture antibody.

The hydrogel elements may each have a volume of no more than 1 mm³, in particular of no more than 0.5 mm³ or of no more than 0.3 mm³.

The hydrogel elements may each have a thickness in a cross section of the capillary tube of no more than 0.4 mm, in particular of no more than 0.3 mm or of no more than 0.2 mm.

Respective embodiments ensure that the hydrogel does not obstruct fluid flow within the capillary tube. This precise thickness allows for efficient interaction with analytes in the fluid.

Maintaining a respective small thickness of the hydrogel elements also ensures that the hydrogel structures are sufficiently thin to allow for rapid diffusion of analytes to the binding sites, thereby enhancing the sensitivity and speed of the assay.

The hydrogel elements may each have a length along a flow direction of the capillary tube of no more than 0.8 mm, in particular of no more than 0.6 mm or of no more than 0.4 mm.

This allows for a more compact design of the analysis device, which is particularly beneficial for multiplexed assays where space and material usage are critical considerations.

Moreover, hydrogel elements with a smaller length, width, thickness, or volume provide a higher surface area-to-volume ratio, facilitating better binding and interaction with target molecules.

According to an embodiment, at least one of the hydrogel elements is, along a flow direction of the capillary tube, spaced apart from an upstream hydrogel element comprising a hydrogel and a binding agent by a first hydrogel element distance, and spaced apart from a downstream neighboring hydrogel element comprising a hydrogel and a binding agent by a second hydrogel element distance, each of the first hydrogel element distance and the second hydrogel element distance being no more than 1 mm, in particular being no more than 0.6 mm or being no more than 0.4 mm.

The hydrogel elements may be spaced apart from each other along a flow direction of the capillary tube.

This configuration facilitates the compartmentalization of different reactions or assays within a single capillary. It also minimizes cross-contamination between different hydrogel elements, thereby improving the accuracy and reliability of the assay results.

The hydrogel elements may have a shape of at least one of a circular hollow cylinder and a cylindrical shell.

Each of the hydrogel elements may have a shape of at least one of a circular hollow cylinder and a cylindrical shell.

Respective embodiments ensure a sufficiently large interface between the hydrogen elements and the inner surface of the capillary tube to achieve a strong enough adhesion to avoid detachment of the hydrogel elements from the inner surface of the capillary tube. At the same time, respective embodiments provide an inner surface of the circular hollow cylinder, or of the cylindrical shell respectively, sufficiently large to ensure efficient absorption of the fluid into the hydrogel elements.

The hydrogel elements may extend along the inner surface of the capillary tube along a circumferential direction of the inner surface of the capillary tube. Each of the hydrogel elements may extend along a corresponding section of the inner surface of the capillary tube along a circumferential direction of the inner surface of the capillary tube.

Respective embodiments ensure a sufficiently large interface between the hydrogen elements and the inner surface of the capillary tube to achieve a strong enough adhesion to avoid detachment of the hydrogel elements from the inner surface of the capillary tube.

According to an aspect of the disclosure, an analysis device is provided. The analysis device comprises a capillary tube. The analysis device comprises hydrogel elements on an inner surface of the capillary tube. The hydrogel elements comprise a covalently cross-linked hydrogel, and a binding agent for immobilizing analytes from a fluid. Each of the hydrogel elements surrounds a portion of an inner cross-sectional area of the capillary tube.

The analysis device and the elements and properties thereof achieve the technical effects and advantages described above in the context of the analysis device fabricated using the disclosed method.

The analysis device may exhibit one or a combination of or all the features of the analysis device described above in the context of the method. To avoid repetition, the respective features will not be described again.

The capillary tube may exhibit one or a combination of or all the features of the capillary tube described above in the context of the method. To avoid repetition, the respective features will not be described again.

The hydrogel elements may exhibit one or a combination of or all the features of the hydrogel elements described above in the context of the method. To avoid repetition, the respective features will not be described again.

The cross-linked hydrogel may exhibit one or a combination of or all the features of the cross-linked hydrogel described above in the context of the method. To avoid repetition, the respective features will not be described again.

The portion of the inner cross-sectional area of the capillary tube may exhibit one or a combination of or all the features of the portion of the inner cross-sectional area of the capillary tube described above in the context of the method. To avoid repetition, the respective features will not be described again.

The portion of the inner cross-sectional area of the capillary tube may be adapted to provide a section of a flow channel through the capillary tube.

The flow channel through the capillary tube may extend from one end of the capillary tube to an opposite end of the capillary tube.

In the context of this disclosure, the flow channel through the capillary tube may refer to the passage within the capillary tube through which fluids can move from one end of the capillary tube to the other end of the capillary tube. This feature ensures that the entire length of the capillary tube is utilized for fluid flow, allowing for continuous and unobstructed movement of the sample or reagents through the capillary tube.

The flow channel also ensures that the hydrogel elements, and if present, the additional hydrogel elements, are accessible for the fluid with the analytes.

The hydrogel elements may be in liquid communication with the flow channel.

The additional hydrogel elements may be in liquid communication with the flow channel.

The analysis device may further comprise additional hydrogel elements on the inner surface of the capillary tube, wherein the additional hydrogel elements comprise a hydrogel and an additional binding agent, and wherein the additional binding agent is adapted to immobilize an additional analyte from the fluid, the additional analyte being different from the analyte.

The additional hydrogel elements may exhibit one or a combination of or all the features of the additional hydrogel elements described above in the context of the method. To avoid repetition, the respective features will not be described again.

The additional hydrogel elements achieve the technical effects and advantages as the additional hydrogel elements described above in the context of the method.

The hydrogel comprised in the additional hydrogel elements may be a cross-linked hydrogel.

The covalently cross-linked hydrogel comprised in the additional hydrogel elements may have a same material composition as the covalently cross-linked hydrogel comprised in the hydrogel elements.

Alternatively, the hydrogel comprised in the additional hydrogel elements may have a material composition different from a material composition of the covalently cross-linked hydrogel comprised in the hydrogel elements.

The application of the covalently cross-linked hydrogel has the advantages described above in the context of the method.

Each of the additional hydrogel elements may surround an additional portion of the inner cross-sectional area of the capillary tube.

For any one of the hydrogel elements and any one of the additional hydrogel elements, the hydrogel element and the additional hydrogel element may be spaced apart from each other along a flow direction of the capillary tube.

Respective embodiments allow for the creation of distinct reaction compartments within the capillary tube, reducing the risk of cross-contamination between different hydrogel elements. This separation also facilitates the analysis of multiple analytes in a single capillary tube, enhancing the multiplexing capability of the device.

The hydrogel elements and the additional hydrogel elements may be arranged intermittently along a flow direction of the capillary tube.

At least some of the additional hydrogel elements may be arranged downstream of the hydrogel elements along a flow direction of the capillary tube.

All of the additional hydrogel elements may be arranged downstream of the hydrogel elements along a flow direction of the capillary tube.

The hydrogel elements may be at least three hydrogel elements or may be at least five hydrogel elements.

According to an embodiment, the analysis device further comprises end elements adapted to close opposing ends of the capillary tube.

Respective end elements serve to reduce or avoid evaporation of the fluid to be analyzed and or of the analyte(s) from the analysis device, more specifically from the hydrogel elements (and/or, in embodiments with the additional hydrogel elements, from the additional hydrogel elements) and ultimately from the capillary tube. Reducing or avoiding evaporation establishes more constant and controlled conditions during an analysis performed using the analysis device, thus improving the accuracy of the result of the analysis.

According to an embodiment, at least one of the end elements comprises or is at least one of: an end cap, a liquid film (in particular, an oil film), and a film of an elastic material.

At least one of the end elements may be detachably connected to one of the opposing ends of the capillary tube.

A further aspect of the invention refers to a use of the analysis device according to any of the embodiments described above for detecting analytes from a fluid, said fluid being arranged in the hydrogel elements.

In said use, the analytes from the fluid may be detected using a bioassay or an immunoassay using the binding agent.

In said use, the hydrogel elements may be separated by a gas or by vacuum while the analytes from the fluid are detected.

In said use, the hydrogel elements may be separated by at least one of the following: air or an inert gas.

The inventors have found that, surprisingly, the analysis device according to this disclosure provides a signal enhancement during the performed essay, such as a bioassay or an immunoassay, in the presence of gas such as air above the hydrogel elements (or above the additional hydrogel elements, respectively), without the need to introduce an additional liquid such as oil above the hydrogel elements (or above the additional hydrogel elements, respectively).

In said use, opposing ends of the capillary tube may be closed while the analytes from the fluid are detected.

According to an aspect of the disclosure, an apparatus for fabricating an analysis device comprising a capillary tube and hydrogel elements on an inner surface of the capillary tube is provided. The device comprises a parallel-flow provider device adapted to provide, at an outlet of the parallel-flow provider device, parallel flows of fluids for introduction into the capillary tube. The parallel-flow provider device comprises, at the outlet of the parallel-flow provider device, a connector for detachably connecting, for the introduction of the parallel flows of fluids into the capillary tube, the capillary tube to the outlet of the parallel-flow provider device. The device comprises a valve adapted for stopping the parallel flows of fluids. The device comprises a lithographic mask. The connector and the lithographic mask are adapted such that, when the capillary tube is connected to the connector and when blue or UV light is provided through the lithographic mask, at least a portion of the blue or UV light illuminates the capillary tube through the lithographic mask.

The apparatus is thus adapted to carry out the method described above, giving the advantages described above in the context of the method.

According to an embodiment, the connector is adapted to permit the parallel flows of fluids to flow through the connector towards the capillary tube.

The connector may be adapted to connect to a first end of the capillary tube.

The connector may comprise or may be composed of an elastomer.

Connecting the connector to a first end of the capillary tube provides a stable and consistent point of entry for the fluids. This design minimizes the risk of leaks or misalignment, thereby ensuring accurate and efficient delivery of fluids into the capillary tube for subsequent processing.

The apparatus may further comprise an additional connector adapted to connect, when the connector is connected to the first end of the capillary tube, to a second end of the capillary tube opposite to the first end of the capillary tube.

This additional connector ensures that the capillary tube can be securely held in place from both sides, facilitating the introduction and flow of fluids through the tube. This ensures that the tube remains aligned and properly positioned. Additionally, it allows for a more controlled and consistent flow of fluids through the tube, enhancing the precision and reliability of the apparatus and the fabrication.

The additional connector may be adapted to permit the parallel flows of fluids to flow through the additional connector out of the capillary tube.

In a further implementation of the apparatus, a horizontal reference line connects the connector and the additional connector and the lithographic mask is arranged below the horizontal reference line along a vertical direction perpendicular to the horizontal reference line.

The apparatus may further comprise a radiation source adapted to provide the blue or UV light.

The radiation source may be arranged below the lithographic mask along the vertical direction perpendicular to the horizontal reference line.

The parallel flows of fluids may comprise, according to a cross section at the outlet of the parallel-flow provider device, a first flow of a first fluid, the first flow of the first fluid being an outermost one of the parallel flows of fluids.

The parallel flows of fluids of the method may be configured accordingly.

The apparatus may further comprise a fluid reservoir in selective liquid communication with the parallel-flow provider device and adapted to selectively provide the first fluid to the parallel-flow provider device for providing the first flow of the first fluid, the fluid reservoir containing the first fluid, the first fluid comprising a hydrogel precursor and a binding agent for immobilizing an analyte from a fluid.

According to some embodiments, the parallel flows of fluids comprise, according to the cross section at the outlet of the parallel-flow provider device, a second flow of a second fluid, wherein the outermost flow of the first fluid surrounds at least a portion of the second flow of the second fluid.

In the context of this disclosure, blue or UV light may comprise at least one wavelength in a wavelength range from 320 nm to 500 nm.

### LIST OF FIGURES

In the following, a detailed description of the present disclosure and examples thereof is given with reference to the figures, wherein
Fig. 1 schematically illustrates a method for fabricating an analysis device;
Fig. 2a and Fig. 2b illustrate the process step of providing parallel flows of fluids in the capillary tube, according to some embodiments, in further detail;
Fig. 3a and Fig. 3b illustrate the process step of stopping the parallel flows of fluids in the capillary tube, according to some embodiments, in further detail;
Fig. 4a and Fig. 4b illustrate the process step of providing blue or UV light in the capillary tube, according to some embodiments, in further detail;
Fig. 5a illustrates an optional method step of providing subsequent parallel flows of fluids in the capillary tube;
Fig. 5b illustrates an optional method step of providing, through a subsequently-applied lithographic mask, blue or UV light in the capillary tube to photopolymerize at least a portion of a subsequently-applied hydrogel precursor comprised in a first subsequent fluid of a first subsequent flow from the subsequent parallel flows of fluids;
Fig. 5c illustrates an optional method step of moving at least one of the capillary tube and the lithographic mask such that the lithographic mask moves from a first position to a second position;
Fig. 6 illustrates an optional method step of silanizating the inner surface of the capillary tube;
Fig. 7 illustrates an optional method step of forming hydrogel elements on an interlayer on the inner surface of the capillary tube;
Fig. 8a and Fig. 8b illustrate an analysis device according to an embodiment;
Fig. 9 schematically illustrates an apparatus for fabricating an analysis device;
Fig. 10 schematically illustrates a parallel-flow provider device of an analysis device according to an embodiment;
Fig. 11 schematically illustrates results of a bioassay performed using an analysis device according to an embodiment;
Fig. 12a to Fig. 12d schematically illustrate the use of an analysis device according to an embodiment; and
Fig. 13a to Fig. 13d schematically illustrate the use of an analysis device according to another embodiment.

### DESCRIPTION OF EXAMPLES

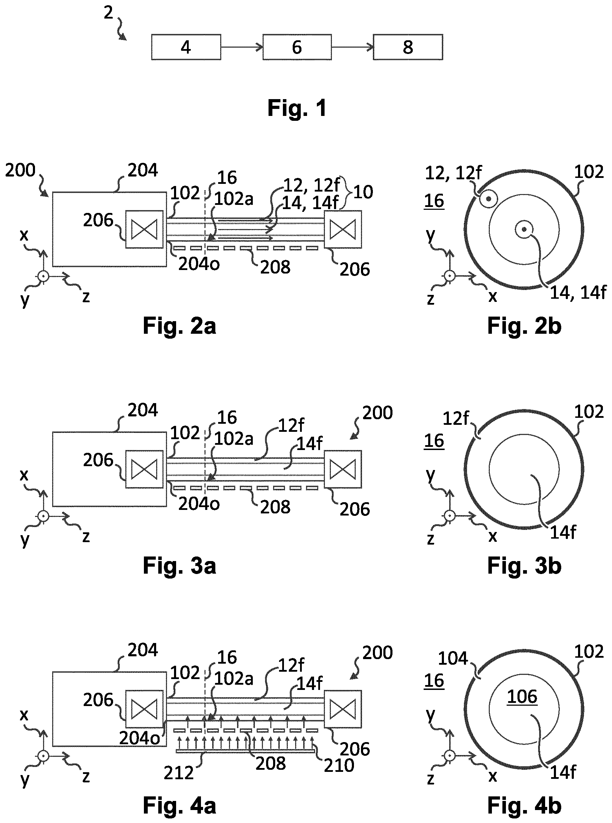

Fig. 1 illustrates a method 2 for fabricating an analysis device 100. The method comprises: providing 4 parallel flows of fluids 10, stopping 6 the parallel flows of fluids 10, and providing 8 blue or UV light 210 through a lithographic mask 208 to photopolymerize at least a portion of the hydrogel precursor.

Fig. 2a to Fig. 4b illustrate a method 2 for fabricating an analysis device 100 in more detail, according to an exemplary embodiment. Fig. 2a, Fig. 3a, and Fig. 4a each give a side view of the method 2, or of an apparatus 200 applied in the method 2, respectively, as viewed from the y direction of the indicated coordinate system. Fig. 2b, Fig. 3b, and Fig. 4b give cross sections corresponding to the x-y-plane through the side views of Fig. 2a, Fig. 3a, and Fig. 4a, as indicated by the plane 16 in Fig. 2a to Fig. 4b.

According to the depicted embodiment, the method 2 uses the apparatus 200 for fabricating the analysis device 100.

The apparatus 200 comprises a parallel-flow provider device 204, at least one valve 206, and a lithographic mask 208.

The parallel-flow provider device 204 comprises two inlets 218i, 220i, and internal channels for merging streams of fluids provided at the inlets 218i, 220i. The parallel-flow provider device 204 generates at its outlet 204o parallel flows of fluids 10, i.e., parallel flows of the fluids provided at the inlets 218i, 220i.

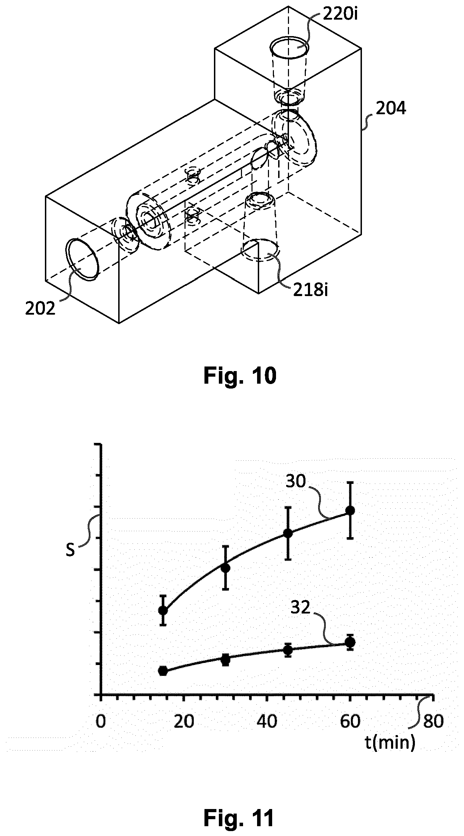

Prototypical parallel-flow provider devices 204 applied by the inventors are made of at least one polymer and fabricated by 3D printing. An exemplary parallel-flow provider device 204 will be described in more detail below in the context of Fig. 10.

A capillary tube 102 is connected to the parallel-flow provider device 204 at an outlet 204o of the parallel-flow provider device 204. More specifically, the capillary tube 102 is connected to the outlet 204o of the parallel-flow provider device 204 such that it can receive at one of its ends 102e the parallel flows of fluids 10, when they are generated by the parallel-flow provider device 204.

The capillary tube 102 has an inner surface 102a.

The apparatus 200 further comprises a lithographic mask 208 arranged below the capillary tube 102. The lithographic mask 208 comprises slits for passing blue or UV light 210 therethrough. The lithographic mask 208 is arranged with the slits perpendicular to a flow direction z of the capillary tube 102. More specifically, in the depicted embodiment, the slits extend along the y direction.

According to different embodiments, the lithographic mask 208 is a photomask, a stencil, or a patterned film.

The apparatus 200 further comprises valves 206 adapted to stop the parallel flows of fluids 10. For this purpose, in the depicted embodiment, a valve 206 is provided upstream of the capillary tube 102, and a valve 206 is provided downstream of the capillary tube 102. In alternative embodiments (not shown) only one of the valves 206 is provided.

According to the depicted example, the capillary tube 102 has a circular cross-section with an inner diameter of 580 micrometres. Other diameters are possible, and other cross-sectional shapes than a circular one are possible. Preferably, the cross section and/or the cross-sectional area are such that the capillary tube 102 promotes a capillary action on aqueous liquids, such as on water.

As shown in Fig. 2a and Fig. 2b, parallel flows of fluids 10 are provided in the capillary tube 102.

More specifically, fluids are provided at the inlets 218i, 220i of the parallel-flow provider device 204, merged in the internal channels of the parallel-flow provider device 204, and provided at the outlet 204o of the parallel-flow provider device 204 as parallel flows of fluids 10.

During the depicted process step of providing 4 the parallel flows of fluids 10 in the capillary tube 102, any of the at least one valves 206 (e.g., the two valves 206 according to the depicted embodiment) is open to allow for the parallel flows of fluids 10 to be provided.

During the depicted process step of providing 4 the parallel flows of fluids 10 in the capillary tube 102, a radiation source 212 (not shown in Fig. 2a, Fig. 2b) adapted to provide blue or UV light 210, through the lithographic mask 208, in the capillary tube 102 is switched off.

At least one of the parallel flows of fluids 10 flows along the inner surface 102a of the capillary tube 102, or is provided on the inner surface 102a of the capillary tube 102, respectively. In the context of this disclosure, the respective one of the parallel flows of fluids 10 is referred to as a first flow 12 of a first fluid 12f.

The first fluid 12f comprises a precursor adapted to form a hydrogel therefrom, which is also referred to as a hydrogel precursor. More specifically, the precursor is a photochemical precursor adapted to form a hydrogel therefrom by photopolymerization of the photochemical precursor under illumination with blue or UV light 210.

In the depicted embodiment, the hydrogel precursor is poly(ethylene glycol) diacrylate, which is also referred to as PEGDA in the context of this disclosure.

According to alternative embodiments, the hydrogel precursor comprises at least one of, or is one of: Allyl Methacrylate; Benzyl Methylacrylate; 1,3-Butanediol Dimethacrylate; 1,4-Butanediol Dimethacrylate 745 Butyl Acrylate n-Butyl Methacrylate; Diethyleneglycol Diacrylate; Diethyleneglycol Dimethacrylate; Ethyl Acrylate 750 Ethyleneglycol Dimethacrylate; Ethyl Methacrylate; 2-Ethyl Hexyl Acrylate; 1,6-Hexanediol Dimethacrylate; 4-Hydroxybutyl Acrylate 755 Hydroxyethyl Acrylate; 2-Hydroxyethyl Methacrylate; 2-Hydroxypropyl Acrylate; Isobutyl Methacrylate; Lauryl Methacrylate 760 Methacrylic Acid; Methyl Acrylate; Methyl Methacrylate; Monoethylene Glycol; 2,2,3,3,4,4,5,5-Octafluoropentyl Acrylate 765 Pentaerythritol Triacrylate; Polyethylene Glycol (200) Diacrylate; Polyethylene Glycol (400) Diacrylate; Polyethylene Glycol (600) Diacrylate; Polyethylene Glycol (200) Dimethacrylate 770 Polyethylene Glycol (400) Dimethacrylate; Polyethylene Glycol (600) Dimethacrylate; Stearyl Methacrylate; Triethylene Glycol; Triethylene Glycol Dimethacrylate 775 2,2,2-Trifluoroethyl 2-methylacrylate; Trimethylolpropane Triacrylate; Acrylamide; N,N-methylene-bisacryl-amide; Phenyl acrylate 780 Divinyl benzene.

In the depicted embodiment, the first fluid 12f further comprises a photoinitiator adapted to accelerate the photopolymerization of the photochemical precursor under illumination with blue or UV light 210.

In the depicted embodiment, the photoinitiator is 2-hydroxy-2-methylpropiophenone, which is also referred to as Darocur 1173 in the context of this disclosure.

In the depicted embodiment, the first fluid 12f further comprises a solvent.

In the depicted embodiment, the solvent is water, more specifically, deionized water.

In the depicted embodiment, the first fluid 12f comprises 55 weight % PEGDA, 40 weight % of the solvent in the exemplary form of the deionized water, and 5 weight % Darocur 1173.

The parallel flows of fluids 10 further comprise a second flow 14 of a second fluid 14f.

In the depicted embodiment, the second flow 14 of the second fluid 14f is encircled by the first flow 12, i.e., according to the cross-section through the capillary tube 102 shown in Fig. 2b, the first flow 12 surrounds the second flow 14 on all sides. In other words, according to the cross-section through the capillary tube 102 shown in Fig. 2b, a closed curve through the first flow 12 exists, which fully encloses the second flow 14.

However, this is not necessarily the case. According to alternative embodiments (not shown), the first flow 12 and the second flow 14 are arranged side-by-side.

According to alternative embodiments (not shown) wherein the first flow 12 surrounds a portion of the second flow 14, the first flow 12 is arranged on two sides of the second flow 14, for example along two sides according to a rectangular coordinate system such as the rectangular coordinate system shown in Fig. 2a to Fig. 4b.

According to alternative embodiments (not shown) wherein the first flow 12 surrounds a portion of the second flow 14, the first flow 12 is arranged on three sides of the second flow 14, for example along three sides according to a rectangular coordinate system such as the rectangular coordinate system shown in Fig. 2a to Fig. 4b.

The second fluid 14f is adapted to be photopolymerized, when it is provided with a same blue or UV light 210 as the first fluid 12f, to a lesser degree than the first fluid 12f.

For this purpose, the second fluid 14f comprises less of a hydrogel precursor than the first fluid 12f.

Alternatively, or in addition, the second fluid 14f comprises less of a photoinitiator than the first fluid 12f.

In the depicted embodiment, the second fluid 14f does not comprise the hydrogel precursor, but comprises the photoinitiator.

The composition of the second fluid 14f is such that it has a density similar to the density of the first fluid 12f.

A similar density of the first fluid 12f and the second fluid 14f beneficially promotes a reliable formation of parallel flows 12, 14 of the fluids 12f, 14f.

The composition of the second fluid 14f is such that it is miscible with the first fluid 12f.

Miscibility of the first fluid 12f and the second fluid 14f beneficially promotes a reliable formation of parallel flows 12, 14 of the fluids 12f, 14f.

The second fluid 14f preferably contains a solvent also contained in the first fluid 12f. This improves miscibility of the fluids 12f, 14f.

In the depicted embodiment, the second fluid 14f comprises 55 weight % Poly(ethylene glycol) dimethacrylate (which is also referred to as PEG in the context of this disclosure), also known as MW 200, 40 weight % of a solvent in the exemplary form of deionized water, and 5 weight % Darocur 1173.

With this composition, the second fluid 14f contains less of the hydrogel precursor than the first fluid 12f. Notably, PEGDA functions as a hydrogel precursor, whereas PEG does not. More specifically, PEGDA is suitable for photopolymerization.

With this composition, the second fluid 14f has a density similar to the one of the first fluid 12f.

With this composition, the second fluid 14f contains a solvent, namely water, and more specifically, deionized water, also contained in the first fluid 12f.

As shown in Fig. 3a and Fig. 3b, the parallel flows of fluids 10 are stopped in the capillary tube 102.

For this purpose, at least one of the valves 206 is closed.

In the depicted embodiment, a valve 206 upstream of the capillary tube 102, or upstream of the outlet 204o of the parallel-fluid provider device 204, respectively, is closed, and a valve 206 downstream of the capillary tube 102, or downstream of the outlet 204o of the parallel-fluid provider device 204, respectively, is closed.

In alternative embodiments (not shown), only one or at least one of a valve 206 upstream of the capillary tube 102, or upstream of the outlet 204o of the parallel-fluid provider device 204, respectively, and a valve 206 downstream of the capillary tube 102, or downstream of the outlet 204o of the parallel-fluid provider device 204, respectively, is closed.

In the depicted embodiment, the valve 206 upstream of the capillary tube 102 is physically comprised in the parallel-fluid provider device 204. However, this is not necessarily the case. In alternative embodiments (not shown), the valve 206 upstream of the capillary tube 102, or upstream of the outlet 204o of the parallel-fluid provider device 204, respectively, is arranged upstream of the parallel-fluid provider device 204, for example, upstream of the inlets 218i, 220i of the parallel-fluid provider device 204.

The stopping of the parallel flows of fluids 10 in the capillary tube 102 results in a static distribution of the first fluid 12f and the second fluid 14f. However, it is clear that the first fluid 12f and the second fluid 14f are not necessarily perfectly static when the parallel flows of fluids 10 in the capillary tube 102 are stopped. Some degree of movement may remain.

For example, the stopped parallel flows of the fluids 10 in the capillary tube 102 may be characterized by a velocity of the first fluid 12f being smaller than a velocity of the first fluid 12f in the first flow 12 of the first fluid 12f at least by a factor of two, or at least by a factor of four, or at least by a factor of 10.

Optionally, the stopped parallel flows of the fluids 10 in the capillary tube 102 may further be characterized by a velocity of the second fluid 14f being smaller than a velocity of the second fluid 14f in the second flow 14 of the second fluid 14f at least by a factor of two, or at least by a factor of four, or at least by a factor of 10.

As shown in Fig. 4a and Fig. 4b, blue or UV light 210 is provided, through a lithographic mask 208, in the capillary tube 102. This photopolymerizes at least a portion of the hydrogel precursor comprised in the first fluid 12f, thereby forming, on the inner surface 102a of the capillary tube 102, the hydrogel elements 104 comprising the hydrogel and the binding agent.

Walls of the capillary tube 102a are at least partially transparent to the blue or UV light 210.

In the context of this disclosure, blue or UV light 210 refers to electromagnetic radiation in the wavelength range from 320 nm to 500 nm. For example, the blue or UV light 210 may be defined as electromagnetic radiation comprising at least one wavelength in the wavelength range from 320 nm to 500 nm.

To provide the blue or UV light 210, a radiation source 212 comprised in the apparatus 200 and adapted to provide the blue or UV light 210 is switched on.

The radiation source 212 can be a laser, a LED, an arc lamp or an incandescent bulb, or any other type of light-emitting device capable of producing the required wavelength of light.

As shown in the depicted embodiment, the lithographic mask 208 and the radiation source 212 are arranged such that the lithographic mask 208 is located between the radiation source 212 and a space for the capillary tube 102.

Said space for the capillary tube 102 is typically next to the outlet 204o of the parallel-flow provider device 204.

More specifically, the space for the capillary tube 102 is offset from the outlet 204o of the parallel-flow provider device 204 along a horizontal direction z. The lithographic mask 208 is arranged under the space for the capillary tube 102 along a vertical direction x. The radiation source 212 is arranged below the lithographic mask 208 along the vertical direction x.

In the context of this disclosure, the terms "horizontal", "vertical", "above", "below" indicate the positions of elements relative to each other. They do not imply a positioning or an orientation relative to an external reference frame, such as an external reference frame defined by the gravitational field of the earth.

In some embodiments, the apparatus 200 comprises a connector (also referred to as an additional connector 222 in the context of this disclosure) for connecting the downstream end of the capillary tube 102 thereto and to support the capillary tube 102 at its downstream end. In the depicted embodiment, the valve 206 located downstream from the capillary tube 102 is in liquid communication with this connector 222. In respective embodiments, the space for the capillary tube 102 can be characterized by a horizontal line interconnecting the outlet 204o of the parallel-flow provider device 204, or a connector located at the outlet 204o of the parallel-flow provider device 204, respectively and the additional connector 222 for connecting to the downstream end of the capillary tube 102.

In the depicted embodiment, since the second fluid 14f does not comprise the hydrogel precursor, the second fluid 14f, although illuminated with the blue or UV light 210 at process step 8, is not photopolymerized and/or converted into a hydrogel. However, this does not mean that no hydrogel at all may be formed at the position of the second flow 14 indicated in Fig. 2a, Fig. 2b. According to some examples, some degree of unavoidable intermixing may occur between the first fluid 12f and the second fluid 14f, and thus, even if the second fluid 14f is provided into the parallel-flow provider device 204 or into the capillary tube 102 without containing any hydrogel precursor, the intermixing may bring some of the hydrogel precursor initially contained in the first fluid 12f to the position of the second flow 14 indicated in Fig. 2a, giving rise to the formation of a minor amount of hydrogel at the position of the second flow 14 indicated in Fig. 2a.

The resulting hydrogel elements 104 each surround a portion 106 of an inner cross-sectional area of the capillary tube 102, as can best be seen in Fig. 4b. This is even the case if some amount of hydrogel is formed at the position of the second flow 14 indicated in Fig. 2a, as discussed in the foregoing paragraph.

In other words, the analysis device 100 is formed with a portion 106 of the inner cross-sectional area of the capillary tube 102 not containing hydrogel.

In yet other words, the portion 106 of the inner cross-sectional area of the capillary tube 102 serves to provide an aperture, or a channel through the capillary tube 102, allowing fluid to pass (flow) from one end of the capillary tube 102 to the opposite end of the capillary tube 102. This is beneficial for introducing fluid into the analysis device 100, or into the capillary tube 102 and to the hydrogel elements 104, respectively, for performing an analysis such as a bioassay or an immunoassay on the fluid.

The method and the apparatus are not limited to the provision of exactly two (flows of) fluids. According to alternative embodiments (not shown), the parallel-flow provider device 204 can provide additional flows of fluids with varying compositions, either for providing additional portions (e.g., a coating) of the hydrogel elements 104, or to provide a further structured pattern of fluid which is not photopolymerized at process step 8.

According to some embodiments, the valve 206 and the radiation source 212 are adapted to be controlled electronically. In respective embodiments, the apparatus 200 comprises a controller (not shown) adapted to control the valve 206 and the radiation source 212 such that the blue or UV light 210 is provided using the radiation source 212 after the parallel flows of fluids 10 are stopped using the valve 206. Optionally, in respective embodiments, the controller is adapted to control the apparatus 200 to perform any of the process steps according to the method 2.

In the depicted embodiment, the binding agent comprised in the first fluid 12f is a tag. In the depicted embodiment, biotin is used as an exemplary tag. The hydrogel elements 104 are thus formed comprising the tag (e.g., biotin) as a binding agent.

After formation of the hydrogel element 104 as described above, in a further, optional process step (not shown) a solution comprising a capture antibody is introduced into the capillary tube 102. The tag (e.g., biotin) selectively binds the capture antibody, thus forming a binding agent comprising both the tag (e.g., biotin) and the capture antibody.

In other words, after the further, optional process step, the hydrogel elements 104 comprise a binding agent comprising the tag (e.g., biotin) and the capture antibody.

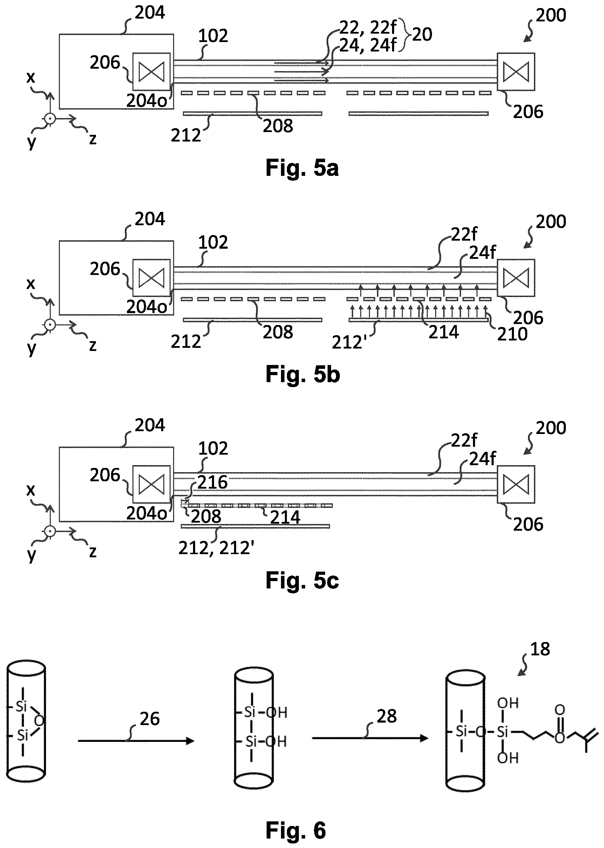

Fig. 5a illustrates a further embodiment of the method 2. More specifically, the illustrated embodiment is similar to the embodiments described above, and Fig. 5a shows an optional, additional process step for introduction after the process steps of the method 2 according to any of the embodiments described above.

More specifically, Fig. 5a illustrates an optional, additional process step of providing subsequent parallel flows of fluids 20 in the capillary tube 102. Said subsequent parallel flows of fluids 20 comprise a first subsequent flow 22 of a first subsequent fluid 22f on the inner surface 102a of the capillary tube 102. The first subsequent fluid 22f comprises a subsequently-applied hydrogel precursor and an additional binding agent, wherein the additional binding agent is adapted to immobilize an additional analyte from the fluid, the additional analyte being different from the analyte.

The first subsequent flow 22 and the first subsequent fluid 22f are similar to the first flow 12 and the first fluid 12f described above in the context of the foregoing embodiments. To avoid repetition, regarding their detailed description, reference is made to the detailed description of the first flow 12 and of the first fluid 12f in the context of the foregoing embodiments.

According to some embodiments, the subsequent parallel flows of fluids 20 comprise a second subsequent flow 24 of a second subsequent fluid 24f, wherein a weight percentage of the subsequently-applied hydrogel precursor is smaller in the second subsequent fluid 24f than in the first subsequent fluid 22f. The second subsequent flow 24 and the second subsequent fluid 24f are similar to the second flow 14 and the second fluid 14f, and for a detailed description of the second subsequent flow 24 and the second subsequent fluid 24f reference is made to the corresponding description of the second flow 14 and the second fluid 14f above.

Optionally, before providing the subsequent parallel flows of fluids 20 in the capillary tube 102 as shown in Fig. 5a, an additional process step (not shown) of rinsing the capillary tube 102 with a rinsing fluid is performed.

Fig. 5b illustrates a further optional process step, which is performed in embodiments of the method according to Fig. 5a after the subsequent parallel flows of fluids 20 are provided in the capillary tube 102. In respective embodiments, a further process step (not shown) of stopping the subsequent parallel flows of fluids 20 in the capillary tube 102 is performed after providing, as illustrated in Fig. 5a, the subsequent parallel flows of fluids 20 in the capillary tube 102. The stopping of the subsequent parallel flows of fluids 20 in the capillary tube 102 is performed similarly to the stopping of the parallel flows of fluids 10 in the capillary tube 102 described above in the context of Fig. 3a. The detailed description of Fig. 3a applies accordingly to the stopping of the subsequent parallel flows of fluids 20 in the capillary tube 102.

As illustrated in Fig. 5b, blue or UV light 210 is provided, through a subsequently-applied lithographic mask 214, in the capillary tube 102 to photopolymerize at least a portion of the subsequently-applied hydrogel precursor comprised in the first subsequent fluid 22f. Thereby additional hydrogel elements 108 are formed on the inner surface 102a of the capillary tube 102. The additional hydrogel elements 108 comprise a subsequently-formed hydrogel and the additional binding agent comprised in the first subsequent fluid 22f. The subsequently-formed hydrogel is formed, using the photopolymerization, from said at least one portion of the subsequently-applied hydrogel precursor.

The provision of the blue or UV light 210 depicted in Fig. 5b is similar to the provision of the blue or UV light 210 depicted in Fig. 4a, and for a detailed description, reference is made to the corresponding description of Fig. 4a above.

Also, the formation of the additional hydrogel elements 108 is similar to the formation of the hydrogel elements 104 described above, and reference is made to the corresponding detailed description above.

The additional hydrogel elements 108 are generally similar to the hydrogel elements 104. However, as opposed to the hydrogel elements 104, which comprise the binding agent described above for selectively immobilizing an analyte, the additional hydrogel elements 108 comprise another binding agent for selectively immobilizing another analyte, different from the analyte that the hydrogel elements 104 are adapted to immobilize.

In other words, the analysis device 100 fabricated using the method according to Fig. 5b comprises hydrogel elements 104 comprising a first binding agent for selectively immobilizing a first analyte, and additional hydrogel elements 108 comprising a second binding agent for selectively immobilizing a second analyte different from the first analyte.

The fabricated analysis device 100 thus allows for, when a fluid comprising different types of analytes is introduced into the analysis device 100, or into the capillary tube 102, respectively, a multiplexed assay (such as a multiplexed bioassay or a multiplexed immunoassay) with different analytes being detected at different positions along the analysis device 100, or along the capillary tube 102, respectively.

The process steps described in the context of Fig. 5a and Fig. 5b are optionally repeated to produce further additional hydrogel elements for detecting further additional analytes at different positions along the analysis device 100, or along the capillary tube 102, respectively.

In Fig. 5a and Fig. 5b a lithographic mask 208 and an additional lithographic mask 214 arranged downstream of the lithographic mask 208 are used in the process steps of photopolymerizing the hydrogel precursor comprised in the first fluid 12f, and in the process steps of photopolymerizing the hydrogel precursor comprised in the first subsequent fluid 22f, respectively.

According to an alternative embodiment, the same lithographic mask 208, 214 is used in the process steps of photopolymerizing the hydrogel precursor comprised in the first fluid 12f and in the process steps of photopolymerizing the hydrogel precursor comprised in the first subsequent fluid 22f.

As illustrated in Fig. 5c, the method according to respective embodiments further comprises the process step of moving 216 at least one of the capillary tube 102 and the lithographic mask 208, 214 such that the lithographic mask 208, 214 moves from a first position (i.e., its position when the hydrogel precursor comprised in the first fluid 12f is photopolymerized) to a second position (i.e., its position when the hydrogel precursor comprised in the first subsequent fluid 22f is photopolymerized).

Respective embodiments of the method are particularly beneficial when the hydrogel elements 104 and the additional hydrogel elements 108 are to be arranged along the analysis device 100, or along the capillary tube 102, respectively, in an intermitting manner. A corresponding movement of the lithographic mask 208, 214 is indicated in Fig. 5c by the arrow 216.

Fig. 6 shows a process for silanizating the inner surface 102a of the capillary tube 102, in embodiments, wherein the inner surface 102a of the capillary tube 102 comprises glass. The process includes hydroxylating 26 the inner surface 102a, followed by applying 28 a silane to the hydroxylated inner surface 102a, resulting in a silanizated inner surface 18.

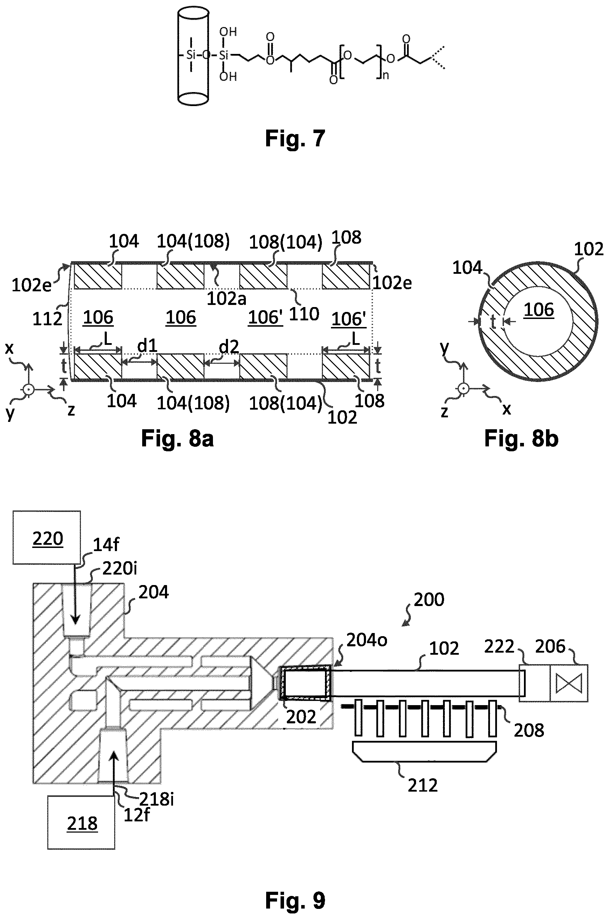

According to alternative embodiments (not shown), the silane molecule may have different functional groups. These functional groups can include amine, epoxy, or thiol groups, depending on the desired chemical properties of the hydrogel elements
Fig. 7 illustrates the binding of the hydrogel of the hydrogel element 104 to the inner surface 102a of the capillary tube 102 during the photopolymerization after the salinization.

The silane molecule depicted in Fig. 7 is used for silanization of the inner surface 102a of the capillary tube 102. The silane molecule has a siloxane backbone with functional groups that can form covalent bonds with the hydroxylated inner surface 102a of the capillary tube 102. This process enhances the adhesion of the hydrogel elements to the inner surface 102a of the capillary tube 102. The silane molecule is typically applied after hydroxylating the inner surface 102a to introduce hydroxyl groups, which react with the silane to form a stable siloxane bond.

The resulting bonding of the hydrogel to the silanized inner surface 102a of the capillary tube 102 ensures reliable adhesion of the hydrogel element 104 to the inner surface 102a of the capillary tube 102.

Fig. 8a depicts a longitudinal cross-sectional view of an analysis device 100 according to an embodiment, and Fig. 8b shows a transverse cross-sectional view of the same analysis device 100.

The capillary tube 102 comprises an inner surface 102a, hydrogel elements 104, and additional hydrogel elements 108.

The arrangement of hydrogel elements 104 and additional hydrogel elements 108 in the capillary tube 102 allows for the simultaneous detection of multiple analytes from a fluid sample. The spacing of the (additional) hydrogel elements 104, 108 ensures efficient fluid flow and interaction with the binding agents, enhancing the sensitivity and specificity of the analysis device 100, and minimizes cross-contamination.

The thickness t of the (additional) hydrogel elements 104, 108 is indicated.

The hydrogel elements 104 surround portions 106 of the inner cross-sectional area of the capillary tube 102.

The additional hydrogel elements 108 surround additional portions 106' of the inner cross-sectional area of the capillary tube 102.

The (additional) hydrogel elements 104, 108 are spaced apart by distances d1 and d2 along the flow direction of the capillary tube 102.

End elements 112 close the opposing ends 102e of the capillary tube 102. In Fig. 8a, only one of the end elements 112 is indicated, the other and element 112 is not shown.

The end elements 112 are detachably connected to the opposing ends 102e of the capillary tube 102 to avoid evaporation of fluid from the hydrogel elements 104 and the additional hydrogel elements 108 during analysis such as an assay, for example a bioassay or an immunoassay. According to the depicted embodiment, the end elements 112 are provided in the form of an oil film on either end 102e of the capillary tube 102. According to alternative embodiments (not shown), the end elements 112 are provided in the form of films of elastic material or as end caps detachably connected to the ends 102e of the capillary tube 102.

Fig. 9 illustrates an apparatus 200 for fabricating an analysis device 100 comprising a capillary tube 102 and hydrogel elements 104 on its inner surface 102a according to an embodiment.

According to the depicted embodiment, the apparatus 200 includes a parallel-flow provider device 204, a valve 206 for stopping the parallel flows of fluids 10, and a lithographic mask 208.

The parallel-flow provider device 204 includes an outlet 204o and a connector 202 for detachably connecting the capillary tube 102 to the outlet 204o.

The parallel-flow provider device 204 comprises inlets 218i, 220i fluid-separated from one another.

The inlets 218i and 220i provide entry points for the fluids 12f, 14f, respectively, to the parallel-flow provider device 204. The inlets 218i and 220i are designed to accommodate tubing or syringes (not shown) that deliver the fluids 12f, 14f. They ensure that the fluids 12f, 14f enter the parallel-flow provider device 204 in a controlled manner, and are thus advantageous for maintaining the parallel flows of fluids 10.

According to some embodiments (not shown), the inlets 218i and 220i are equipped with one-way valves, flow meters, and/or pressure sensors.

The parallel-flow provider device 204 is in selective liquid communication with a fluid reservoir 218, which contains the first fluid 12f. More specifically, the parallel-flow provider device 204 is in selective liquid communication with the fluid reservoir 218 via the inlet 218i.

The parallel-flow provider device 204 is in selective liquid communication with a fluid reservoir 220, which contains the second fluid 14f. More specifically, the parallel-flow provider device 204 is in selective liquid communication with the fluid reservoir 220 via the inlet 220i.

In the depicted embodiment, the connector 202 is a silicone tube 202. The connector 202 in the exemplary form of the silicone tube 202 is connected to a barb at the outlet 204o of the parallel-flow provider device 204. When the parallel flows of fluids 10 reach the outlet 204o of the parallel-flow provider device 204, they pass through the barb and through the silicone tube 202 towards the capillary tube 102.

According to the depicted embodiment, the apparatus comprises a radiation source 212 for providing blue or UV light 210 through the lithographic mask 208.

According to alternative embodiments (not shown), the parallel-flow provider device 204 comprises additional fluid reservoirs for introducing more than two fluids 12f, 14f, allowing for even more complex hydrogel element patterns. The lithographic mask 208 may also have different designs to create various hydrogel element shapes and distributions.

According to the depicted embodiment, the apparatus 200 further comprises an additional connector 222. The additional connector 222 is arranged and adapted such that, when the first end 102e of the capillary tube 102 is connected to the connector 202, the additional connector 222 connects to a second end 102e of the capillary tube 102 opposite to the first end 102e of the capillary tube 102. The additional connector 222 is beneficial for supporting the capillary tube 102 at its second end 102e, improving the mechanical stability of the system and thus the resolution of the (additional) hydrogel elements 104, 108 being produced.

The additional connector 222 is formed similarly to the connector 202 described above.

According to alternative embodiments (not shown), the connector 202 and/or the additional connector 222 comprises or is adapted to provide a threaded or quick-connect fitting, a luer lock fitting, a compression fitting, or a barbed fitting.

Fig. 10 gives a detailed illustration of the parallel-flow provider device 204 of Fig. 9.

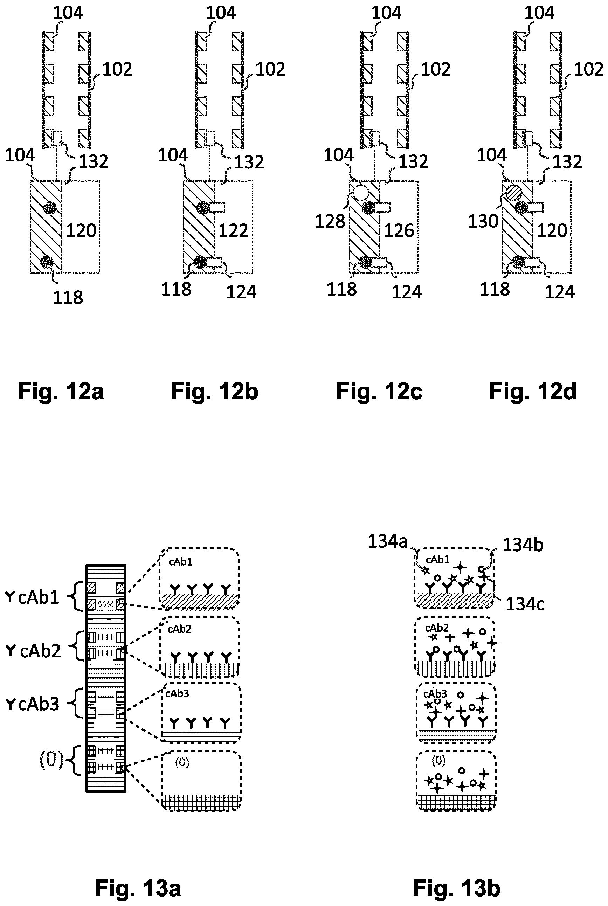

Fig. 11 illustrates a graph showing the relationship between the signal intensity S and time t obtained during an analysis performed using the analysis device 100. A corresponding analysis is given in detail in the context of Fig. 12a to Fig. 12d.

The signal intensity 30 was measured from a hydrogel element 104 contained in an analysis device 100 as described above. The reference signal intensity 32 was measured from a reference hydrogel element also contained in the analysis device 100. The reference hydrogel element is similar to the hydrogel elements 104 described above, but without the binding agent. It is produced similarly as described above in the context of the additional hydrogel element 108, however, without any additional binding agent comprised in the first subsequent fluid 22f.

As the measured signal intensity 30 demonstrates, the analysis device 100, or the hydrogel element 104, respectively, facilitates a signal enhancement in the analysis. This becomes particularly apparent when comparing to the reference signal intensity 32.

Fig. 12a to Fig. 12d illustrate various stages of performing an analysis in the form of a bioassay using the analysis device 100.

The upper panel of any of Fig. 12a to Fig. 12d gives a cross-sectional view of the capillary tube 102 and the hydrogel elements 104. The lower panel shows an enlarged close-up of the section 132 indicated in the upper panel. Molecules 118 of the binding agent, i.e., capture anti-bodies or biotin 118, are indicated in the close-up of section 132. In the depicted example, biotin 118 is indicated.

Fig. 12a shows the capillary tube 102 and the hydrogel elements 104 at the beginning of the bioassay. At the beginning of the bioassay, the inner volume of the capillary tube 102 is filled with air 120.

Subsequently, a fluid 122 comprising an analyte is introduced into the capillary tube 102 using the capillary action thereof.

Fig. 12b shows the capillary tube 102 and the hydrogel elements 104 with the fluid 122 comprising the analyte introduced in the capillary tube 102. In the depicted embodiment, the analyte is Horseradish peroxidase (HRP)-streptavidin 124. The biotin 118 selectively immobilize the HRP-streptavidin 124.

Subsequently, the fluid 122 is removed from the capillary tube 102. In the depicted embodiment, this is achieved using the capillary action of an absorbent pad (not shown) brought into contact with an end 102e of the capillary tube 102.

Subsequently, a solution 126 comprising a detection reagent 128 in the exemplary form of 10-Acetyl-3,7-dihydroxyphenoxazine (ADHP) 128 or a QuantaRed solution 126 is introduced into the capillary tube 102 using the capillary action thereof.

Fig. 12c shows the capillary tube 102 with the respective solution 126 therein.

Right away after the introduction of the solution 126 comprising ADHP 128 or the QuantaRed solution 126 into the capillary tube 102, the solution 126 comprising ADHP 128 or the QuantaRed solution 126 is removed from the capillary tube 102. In the depicted embodiment, this is achieved using the capillary action of an absorbent pad (not shown) brought into contact with an end 102e of the capillary tube 102.

Consequently, the capillary tube 102 is filled with air 120, as depicted in Fig. 12d. in this situation, the HRP-streptavidin 124 promotes the conversion of ADHP 128 from the solution 126 into resorufin 130. Light emission from the resorufin 130 is then detected as the signal S, depicted, for example, in Fig. 11.

The steps shown in Fig. 12a to Fig. 12d follow a known protocol of sandwich ELISA, as previ-ously reported in: Ghulam Destgeer, Mengxing Ouyang, Chueh-Yu Wu, and Dino Di Car-lo: "Fabrication of 3D concentric amphiphilic microparticles to form uniform nanoliter reaction volumes for amplified affinity assays," Lab on a Chip, 2020, 20, 3503-3514. This document is incorporated herein in its entirety by reference.

Fig. 13a and Fig. 13b illustrate the arrangement of different binding agents and antigens within the hydrogel elements 104 and the additional hydrogel elements 108.

Fig. 13a shows a cross-sectional view of the capillary tube 102 with multiple hydrogel elements 104, each containing a different binding agent (cAb1, cAb2, cAb3). These binding agents cAb1, cAb2, cAb3 are used to immobilize different analytes from the fluid. A reference hydrogel element (0) does not comprise any binding agent.

According to an embodiment, the binding agents cAb1, cAb2, cAb3 are capture anti-bodies.

According to an alternative embodiment, the binding agents cAb1, cAb2, cAb3 are nanoparticles coated with capture antibodies.

These binding agents cAb1, cAb2, cAb3 are incorporated into the hydrogel elements 104 during the photopolymerization process.

According to alternative embodiments (not shown), the binding agents cAb1, cAb2, cAb3 comprise other types of molecules, such as aptamers, peptides, or small molecules, depending on the target analytes.

Fig. 13b provides a detailed view of the hydrogel elements 104 when antigens 134a, 134b, and 134c are introduced into the capillary tube 102. The hydrogel elements 104 and the additional hydrogel elements 108 are each provided with a specific binding agent (cAb1, cAb2, cAb3), allowing for the selective binding of the antigens 134a, 134b, and 134c.

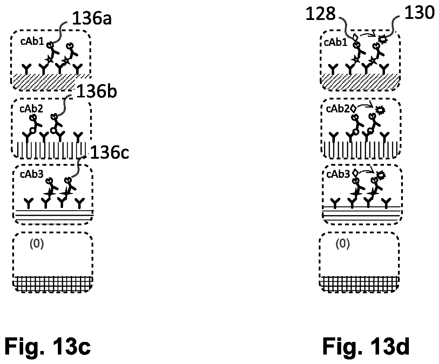

Fig. 13c shows the hydrogel elements 136a, 136b, and 136c after a fluid comprising analytes 136a, 136b, 136c has been introduced to the capillary tube 102. Each of the different binding agents cAb1, cAb2, cAb3 with the antigens 134a, 134b, and 134c attached thereto immobilizes a different one of the analytes 136a, 136b, 136c.

As shown in Fig. 13d, a solution comprising at least one detection reagent 128 is introduced into the capillary tube 102. The analytes 136a, 136b, 136c immobilized at the binding agents cAb1, cAb2, cAb3 with the antigens 134a, 134b, and 134c attached thereto promote the conversion of the detection reagent 128 into the indicator compound 130.

An example for a suitable detection reagent 128, namely, ADHP, and a resulting indicator compound 130, namely, resorufin, is given in the context of Fig. 12c, Fig. 12d. Other suitable systems include:

First example: Biomarker to be detected: Recombinant human IL-6 protein (R&D Systems 206-IL). Capture Anti-bodies: Human IL-6 MAb (Clone 973132) (R&D Systems MAB 9540). Detection Anti-bodies: Human IL-6 antibody (R&D Systems MAB2063).

Second example: Biomarker to be detected: Recombinant human TNF-alpha protein (R&D Systems 210-TA). Capture Anti-bodies: Human TNF-alpha antibody (R&D Systems MAB610). Detection Anti-bodies: Human TNF- alpha antibody (R&D Systems BAF210).

Third example: Biomarker to be detected: Recombinant human IL-8/CXCL8 protein (R&D Systems 208-IL). Capture Anti-bodies: Human IL-8/CXCL8 antibody (R&D Systems MAB2081). Detection Anti-bodies: Human IL-8/CXCL8 antibody (R&D Systems MAB2082).

Fourth example: Biomarker to be detected: Recombinant human C-reactive protein/CRP protein (R&D Systems 1707-CR). Capture Anti-bodies: Human C-reactive protein/CRP antibody (R&D Systems MAB17073). Detection Anti-bodies: Human C-reactive protein/CRP anti-body (R&D Systems MAB17072).

Fifth example: Biomarker to be detected: Recombinant anti-cardiac troponin I (cTnI) (Hytest Finland Cat.# RC4T21). Capture Anti-bodies: Troponin I cardiac (cTnI), antibody (Hytest Finland 4T21/4T21cc, M18cc). Detection Anti-bodies: Troponin I cardiac (cTnI), anti-body (Hytest Finland 4T21/4T21cc, 4C2cc).

Sixth example: Biomarker to be detected: NT-proBNP (HyTest, Finland 8NT2). Capture Anti-bodies: Anti-NT-proBNP (HyTest, Finland 4NT1cc 15C4cc). Detection Anti-bodies: Anti-NT-proBNP (HyTest, Finland 4NT1cc 13G12cc).

The examples of the present disclosure disclosed herein only constitute specific examples for illustration purposes. The present invention can be implemented in various ways and with many modifications without altering the underlying basic properties. Therefore, the present invention is only defined by the claims as stated below.

## Claims

1. A method (2) for fabricating an analysis device (100) comprising a capillary tube (102) and hydrogel elements (104) on an inner surface (102a) of the capillary tube (102), the hydrogel elements (104) comprising a hydrogel and a binding agent for immobilizing an analyte from a fluid, the method (2) comprising:
providing (4) parallel flows of fluids (10) in the capillary tube (102), said parallel flows of fluids (10) comprising a first flow (12) of a first fluid (12f) on the inner surface (102a) of the capillary tube (102), wherein the first fluid (12f) comprises a hydrogel precursor and the binding agent;
stopping (6) the parallel flows of fluids (10) in the capillary tube (102); and
providing (8), through a lithographic mask (208), blue or UV light (210) in the capillary tube (102) to photopolymerize at least a portion of the hydrogel precursor comprised in the first fluid (12f), thereby forming, on the inner surface (102a) of the capillary tube (102), the hydrogel elements (104) comprising the hydrogel and the binding agent.

2. The method (2) according to claim 1,
wherein the parallel flows of fluids (10) comprise a second flow (14) of a second fluid (14f), wherein a weight percentage of the hydrogel precursor is smaller in the second fluid (14f) than in the first fluid (12f);
wherein the parallel flows of fluids (10) are provided in the capillary tube (102) such that, in a cross section (16) of the capillary tube (102), the first flow (12) of the first fluid (12f) surrounds at least a portion of the second flow (14) of the second fluid (14f); and
wherein, in the process step of forming the hydrogel elements (104) comprising the hydrogel and the binding agent, the hydrogel elements (104) are formed to surround a portion (106) of an inner cross-sectional area of the capillary tube (102), said portion of the inner cross-sectional area of the capillary tube (102) corresponding to said at least one portion of the second flow (14) of the second fluid (14f).

3. The method (2) according to claim 2,
wherein the parallel flows of fluids (10) are provided in the capillary tube (102) such that the first flow (12) of the first fluid (12f) surrounds the second flow (14) of the second fluid (14f); and/or
wherein, in the process step of forming the hydrogel elements (104) comprising the hydrogel and the binding agent, the hydrogel elements (104) are formed to encircle the portion of the inner cross-sectional area of the capillary tube (102) ; and/or
wherein the weight percentage of the hydrogel precursor in the second fluid (14f) is smaller than the weight percentage of the hydrogel precursor in the first fluid (12f) at least by a factor of two, or at least by a factor of five, or at least by a factor of ten, or at least by a factor of twenty, and/or wherein the second fluid (14f) does not comprise the hydrogel precursor; and/or
wherein a density of the second fluid (14f) equals a density of the first fluid (12f) within 30% of the density of the first fluid (12f), in particular within 20% of the density of the first fluid (12f) or within 10% of the density of the first fluid (12f); and/or wherein the first fluid (12f) and the second fluid (14f) are adapted to be miscible.

4. The method (2) according to any of the preceding claims, which further comprises, after providing (8) the blue or UV light (210) in the capillary tube (102) to photopolymerize the at least one portion of the hydrogel precursor comprised in the first fluid (12f):
providing subsequent parallel flows of fluids (20) in the capillary tube (102), said subsequent parallel flows of fluids (20) comprising a first subsequent flow (22) of a first subsequent fluid (22f) on the inner surface (102a) of the capillary tube (102),
wherein the first subsequent fluid (22f) comprises a subsequently-applied hydrogel precursor and an additional binding agent, wherein the additional binding agent is adapted to immobilize an additional analyte from the fluid, the additional analyte being different from the analyte;
stopping the subsequent parallel flows of fluids (20) in the capillary tube (102); and providing, through a subsequently-applied lithographic mask (214), blue or UV light (210) in the capillary tube (102) to photopolymerize at least a portion of the subsequently-applied hydrogel precursor comprised in the first subsequent fluid (22f), thereby forming, on the inner surface (102a) of the capillary tube (102),
additional hydrogel elements (108), the additional hydrogel elements (108) comprising a subsequently-formed hydrogel and the additional binding agent,
wherein the subsequently-formed hydrogel is formed, using the photopolymerization, from said at least one portion of the subsequently-applied hydrogel precursor.

5. The method (2) according to claim 4,
wherein the lithographic mask (208) and the subsequently-applied lithographic mask (214) are arranged at different positions along a parallel-flow direction, said parallel-flow direction being the flow direction of the parallel flows of fluids (10) when the parallel flows of fluids (10) are provided in the capillary tube (102); and/or wherein the subsequently-applied lithographic mask (214) is an additional lithographic mask different from the lithographic mask (208); and/or wherein a same lithographic mask is used as the lithographic mask (208) and as the subsequently-applied lithographic mask (214), wherein, in the process step of providing (8) the blue or UV light (210) in the capillary tube (102) through the lithographic mask (208), the same lithographic mask is arranged at a first position relative to the capillary tube (102); wherein, in the process step of providing (8) the blue or UV light (210) in the capillary tube (102) through the subsequently-applied lithographic mask (214), the same lithographic mask is arranged at a second position relative to the capillary tube (102) different from the first position; and wherein the method (2) further comprises, in between the process step of providing (8) the blue or UV light (210) in the capillary tube (102) through the lithographic mask (208) and the process step of providing the blue or UV light (210) in the capillary tube (102) through the subsequently-applied lithographic mask (214), moving (216) at least one of the capillary tube (102) and the same lithographic mask such that the same lithographic mask moves from the first position to the second position;
in particular, wherein the method further comprises, between the process step of providing (8) the blue or UV light (210) in the capillary tube (102) through the lithographic mask (208) and the process step of providing the subsequent parallel flows of fluids (20) in the capillary tube (102), removing the first fluid (12f) from the capillary tube (102);
wherein, optionally, removing the first fluid (12f) from the capillary tube (102) comprises rinsing the capillary tube (102) with a rinsing liquid, wherein a weight percentage of the hydrogel precursor is smaller in the rinsing liquid than in the first fluid (12f); and/or
wherein the subsequent parallel flows of fluids (20) comprise a second subsequent flow (24) of a second subsequent fluid (24f), wherein a weight percentage of the subsequently-applied hydrogel precursor is smaller in the second subsequent fluid (24f) than in the first subsequent fluid (22f); wherein the subsequent parallel flows of fluids (20) are provided in the capillary tube (102) such that the first subsequent flow (22) of the first subsequent fluid (22f) surrounds at least a portion of the second subsequent flow (24) of the second subsequent fluid (24f); and wherein, in the process step of forming the additional hydrogel elements (108) comprising the subsequently-formed hydrogel and the additional binding agent, the additional hydrogel elements (108) are formed to surround a second portion of an inner cross-sectional area of the capillary tube (102), the second portion of the inner cross-sectional area of the capillary tube (102) corresponding to said at least one portion of the second subsequent flow (24) of the second subsequent fluid (24f); and/or wherein the subsequently-formed hydrogel comprises or is a covalently cross-linked hydrogel, and wherein the method (2) further comprises forming the covalently cross-linked hydrogel in the process step of photopolymerizing the at least one portion of the subsequently-applied hydrogel precursor comprised in the first subsequent fluid (22f).

6. The method (2) according to any of the preceding claims,
wherein the hydrogel comprises or is a covalently cross-linked hydrogel, and wherein the method (2) further comprises forming the covalently cross-linked hydrogel in the process step of photopolymerizing the at least one portion of the hydrogel precursor comprised in the first fluid (12f); and/or wherein an inner cross-sectional area of the capillary tube (102) is no larger than 1 mm²; or wherein an inner cross-sectional area of the capillary tube (102) has, along at least one direction, an extension of no more than 1 mm, in particular, wherein the inner cross-sectional area of the capillary tube (102) is no larger than 1 mm²; or wherein an inner cross-sectional area of the capillary tube (102) is circular or elliptical and no larger than 1 mm², wherein, optionally, the inner cross-sectional area of the capillary tube (102) is circular with a diameter of no more than 1 mm; and/or
wherein the hydrogel elements (104), when they are formed on the inner surface (102a) of the capillary tube (102), are formed in direct physical contact with the inner surface (102a) of the capillary tube (102); or wherein the hydrogel elements (104), when they are formed on the inner surface (102a) of the capillary tube (102), are formed on an interlayer such that the interlayer is located between the hydrogel elements (104) and the inner surface (102a) of the capillary tube (102), in particular, wherein the interlayer comprises at least one of a siloxane and a silane layer covalently bonded to the inner surface (102a) of the capillary tube (102); and/or wherein the capillary tube (102) comprises or is composed of glass; in particular, wherein the method (2) further comprises, before the hydrogel elements (104) are formed on the inner surface (102a) of the capillary tube (102), silanizating (18) the inner surface (102a) of the capillary tube (102), and wherein the hydrogel elements (104) are formed on the silanizated inner surface (102a) of the capillary tube (102); wherein, optionally, silanizating (18) the inner surface (102a) of the capillary tube (102) comprises: hydroxylating (26) the inner surface (102a) of the capillary tube (102), and applying (28) a silane to the hydroxylated inner surface (102a) of the capillary tube (102); and/or
wherein the binding agent comprises at least one of the following or is one of the following: a tag adapted to bind a capture antibody thereto, a capture antibody, and nanoparticles coated with a capture antibody; or wherein the binding agent comprises a tag adapted to selectively bind a capture antibody thereto and/or comprises a capture antibody; and/or
wherein the hydrogel elements (104) each have a volume of no more than 1 mm³, in particular of no more than 0.5 mm³ or of no more than 0.3 mm³; and/or wherein the hydrogel elements (104) each have a thickness (t) in a cross section (16) of the capillary tube (102) of no more than 0.4 mm, in particular of no more than 0.3 mm or of no more than 0.2 mm; and/or
wherein the hydrogel elements (104) each have a length (L) along a flow direction of the capillary tube (102) of no more than 0.8 mm, in particular of no more than 0.6 mm or of no more than 0.4 mm; and/or
wherein at least one of the hydrogel elements (104) is, along a flow direction of the capillary tube (102), spaced apart from an upstream hydrogel element comprising a hydrogel and a binding agent by a first hydrogel element distance (d1), and spaced apart from a downstream neighboring hydrogel element comprising a hydrogel and a binding agent by a second hydrogel element distance (d2), each of the first hydrogel element distance (d1) and the second hydrogel element distance (d2) being no more than 1 mm, in particular being no more than 0.6 mm or being no more than 0.4 mm; and/or
wherein the hydrogel elements (104) are spaced apart from each other along a flow direction of the capillary tube (102); and/or
wherein the hydrogel elements (104) have a shape of at least one of a circular hollow cylinder and a cylindrical shell, in particular, wherein each of the hydrogel elements (104) has a shape of at least one of a circular hollow cylinder and a cylindrical shell; and/or
wherein the hydrogel elements (104) extend along the inner surface (102a) of the capillary tube (102) along a circumferential direction of the inner surface (102a) of the capillary tube (102), in particular, wherein each of the hydrogel elements (104) extends along a corresponding section of the inner surface (102a) of the capillary tube (102) along a circumferential direction of the inner surface (102a) of the capillary tube (102).

7. An analysis device (100) comprising a capillary tube (102) and hydrogel elements (104) on an inner surface (102a) of the capillary tube (102),
wherein the hydrogel elements (104) comprise a covalently cross-linked hydrogel and a binding agent for immobilizing analytes from a fluid; and
wherein each of the hydrogel elements (104) surrounds a portion (106) of an inner cross-sectional area of the capillary tube (102).

8. The analysis device (100) according to claim 7,
wherein the portion of the inner cross-sectional area of the capillary tube (102) is adapted to provide a section of a flow channel (110) through the capillary tube (102); in particular, wherein the flow channel (110) through the capillary tube (102) extends from one end (102e) of the capillary tube (102) to an opposite end (102e) of the capillary tube (102).

9. The analysis device (100) according to claim 7 or 8, further comprising additional hydrogel elements (108) on the inner surface (102a) of the capillary tube (102), wherein the additional hydrogel elements (108) comprise a hydrogel and an additional binding agent, and
wherein the additional binding agent is adapted to immobilize an additional analyte from the fluid, the additional analyte being different from the analyte;
in particular:
wherein the hydrogel comprised in the additional hydrogel elements (108) is a cross-linked hydrogel;
wherein, optionally:
the covalently cross-linked hydrogel comprised in the additional hydrogel elements (108) has a same material composition as the covalently cross-linked hydrogel comprised in the hydrogel elements (104); and/or the hydrogel comprised in the additional hydrogel elements (108) has a material composition different from a material composition of the covalently cross-linked hydrogel comprised in the hydrogel elements (104);
and/or
wherein the hydrogel comprised in the additional hydrogel elements (108) has a material composition different from a material composition of the covalently cross-linked hydrogel comprised in the hydrogel elements (104);
and/or
wherein each of the additional hydrogel elements (108) surrounds an additional portion (116') of the inner cross-sectional area of the capillary tube (102); and/or
wherein, for any one of the hydrogel elements (104) and any one of the additional hydrogel elements (108), the hydrogel element and the additional hydrogel element (108) are spaced apart from each other along a flow direction of the capillary tube (102); and/or
wherein the hydrogel elements (104) and the additional hydrogel elements (108) are arranged intermittently along a flow direction of the capillary tube (102); and/or
wherein at least some of the additional hydrogel elements (108) are arranged downstream of the hydrogel elements (104) along a flow direction of the capillary tube (102); and/or
wherein all of the additional hydrogel elements (108) are arranged downstream of the hydrogel elements (104) along a flow direction of the capillary tube (102).

10. The analysis device (100) according to any of claims 7 to 9,
wherein the hydrogel elements (104) are at least three hydrogel elements (104) or at least five hydrogel elements (104); and/or
wherein the analysis device (100) further comprises end elements (112) adapted to close opposing ends (102e) of the capillary tube (102); in particular, wherein at least one of the end elements (112) is detachably connected to one of the opposing ends (102e) of the capillary tube (102).

11. Use of the analysis device (100) according to any of claims 7 to 10 for detecting analytes from a fluid, said fluid being arranged in the hydrogel elements (104).

12. The use according to claim 11,
wherein the analytes from the fluid are detected using a bioassay or an immunoassay using the binding agent; and/or
wherein the hydrogel elements (104) are separated by a gas or by vacuum while the analytes from the fluid are detected; and/or
wherein the hydrogel elements (104) are separated by at least one of the following:
air or an inert gas; and/or
wherein opposing ends (102e) of the capillary tube (102) are closed while the analytes from the fluid are detected.

13. An apparatus (200) for fabricating an analysis device (100) comprising a capillary tube (102) and hydrogel elements (104) on an inner surface (102a) of the capillary tube (102), the device comprising:
a parallel-flow provider device (204) adapted to provide, at an outlet (204o) of the parallel-flow provider device (204), parallel flows of fluids (10) for introduction into the capillary tube (102);
wherein the parallel-flow provider device (204) comprises, at the outlet (204o) of the parallel-flow provider device (204), a connector (202) for detachably connecting, for the introduction of the parallel flows of fluids (10) into the capillary tube (102), the capillary tube (102) to the outlet (204o) of the parallel-flow provider device (204);
a valve (206) adapted for stopping (6) the parallel flows of fluids (10); and
a lithographic mask (208);
wherein the connector (202) and the lithographic mask (208) are adapted such that, when the capillary tube (102) is connected to the connector (202) and when blue or UV light (210) is provided through the lithographic mask (208), at least a portion of the blue or UV light (210) illuminates the capillary tube (102) through the lithographic mask (208).

14. The apparatus (200) according to claim 13,
wherein the connector (202) is adapted to permit the parallel flows of fluids (10) to flow through the connector (202) towards the capillary tube (102); and/or wherein the connector (202) is adapted to connect to a first end (102e) of the capillary tube (102);
in particular, wherein the apparatus (200) further comprises an additional connector (222) adapted to connect, when the connector (202) is connected to the first end (102e) of the capillary tube (102), to a second end (102e) of the capillary tube (102) opposite to the first end (102e) of the capillary tube (102);
wherein, optionally:
the additional connector (222) is adapted to permit the parallel flows of fluids (10) to flow through the additional connector (222) out of the capillary tube (102); and/or
a horizontal reference line connects the connector (202) and the additional connector (222), and wherein the lithographic mask (208) is arranged below the horizontal reference line along a vertical direction perpendicular to the horizontal reference line; in particular,
wherein the apparatus further comprises a radiation source (212) adapted to provide the blue or UV light (210), wherein the radiation source (212) is arranged below the lithographic mask (208) along the
vertical direction perpendicular to the horizontal reference line;
and/or
wherein the parallel flows of fluids (10) comprise, according to a cross section (16) at the outlet (204o) of the parallel-flow provider device (204), a first flow (12) of a first fluid (12f), the first flow (12) of the first fluid (12f) being an outermost one of the parallel flows of fluids (10); in particular:
wherein the apparatus (200) further comprises a fluid reservoir in selective liquid communication with the parallel-flow provider device (204) and
adapted to selectively provide the first fluid (12f) to the parallel-flow provider device (204) for providing (4) the first flow (12) of the first fluid (12f), the fluid reservoir containing the first fluid (12f), the first fluid (12f) comprising a hydrogel precursor and a binding agent for immobilizing an analyte from a fluid; and/or
wherein the parallel flows of fluids (10) comprise, according to the cross section at the outlet (204o) of the parallel-flow provider device (204), a second flow (14) of a second fluid (14f), wherein the outermost flow of the first fluid (12f) surrounds at least a portion of the second flow (14) of the second fluid (14f).

15. The apparatus (200) according to claim 13, further comprising a radiation source (212) adapted to provide the blue or UV light (210).
